# EUROPÄISCHE PATENTSCHRIFT

(11) **EP 3 062 828 B1**
(45) Veröffentlichungstag und Bekanntmachung des Hinweises auf die Patenterteilung: **15.08.2018**
(21) Anmeldenummer: 14827409.5
(22) Anmeldetag: 30.10.2014
(51) Int. Cl.: A61K 51/12, A61K 103/00, A61K 103/20, A61K 103/32, A61P 35/00

(54) **RADIOAKTIVE MIKROSPHÄREN AUS NANOPORÖSEM GLAS FÜR DIE STRAHLENTHERAPIE**
RADIOACTIVE MICROSPHERES MADE OF NANOPOROUS GLASS FOR RADIATION THERAPY
MICROSPHÈRES RADIOACTIVES EN VERRE NANO-POREUX POUR LA RADIOTHÉRAPIE

(30) Priorität: 01.11.2013 DE 102013018685
(43) Veröffentlichungstag der Anmeldung: 07.09.2016
(73) Patentinhaber: Fritz, Eberhard, 13187 Berlin (DE)
(72) Erfinder: Fritz, Eberhard, 13187 Berlin (DE)
(74) Vertreter: Castell, Klaus
(86) Internationale Anmeldenummer: PCT/DE2014/000561
(87) Internationale Veröffentlichungsnummer: WO 2015/062574

(56) Entgegenhaltungen:
- WO-A1-02/34298
- WO-A1-02/34298
- WO-A1-99/51278
- WO-A2-2011/008939
- WO-A2-2011/008939

## Beschreibung

Radioaktive Mikrosphären für die Tumortherapie besitzen eine kugelförmige Geometrie, enthalten ein therapeutisches Radionuklid, vorwiegend Yttrium-90 (Y-90), und kommen bei der Behandlung von nichtoperablen Lebertumoren zur Anwendung. Die Methode ist als Selektive Interne Radiotherapie (SIRT) oder auch als Radioembolisation bekannt.

Weltweit erkranken jährlich mehr als eine Millionen Patienten an Lebertumoren mit überwiegend schlechter Prognose. Die Strahlentherapie mit radioaktiven Mikrosphären verbessert die Lebensqualität und verlängert das Leben der betroffenen Patienten. Es kommen bisher zwei Varianten von Mikrosphären zur Anwendung. Sie unterscheiden sich durch ihre physikalischen Parameter und durch die Art der Herstellung.

[SALEM 2006] gibt einen Überblick über die Anwendung der Mikrosphären der Firmen NORDION (Kanada) und SIRTEX Medical (Australien).

Häfeli hat in einem früheren Review die therapeutische Bedeutung der Mikrosphären zusammengefasst [HÄFELI 2001]. Bei beiden Varianten wird die Betastrahlung des Radionuklides Y-90 therapeutisch angewendet.

NORDION (Therasphere®) erzeugt das Y-90 durch Neutronenaktivierung im Kernreaktor aus nicht radioaktivem Y-89, das im Glasherstellungsprozess zugesetzt wurde.

NORDION nutzt eine Technologie, die in den Patenten US 4,789,501 und US 5,011,677 (University of Missouri, USA) offengelegt wurde. Die Neutronenaktivierung erzeugt jedoch nicht ausschließlich das Radionuklid Y-90 im Glas der Mikrosphären sondern auch weitere Radionuklide, die bei der Therapie unerwünscht und teilweise auch schädlich sind. Dieser Umstand kann durch eine längere Wartezeit zwischen der Neutronenaktivierung und der Therapie zwar abgeschwächt werden, ist aber nicht völlig vermeidbar.

Der aktuelle Kenntnisstand zur Therapie mit Therasphere® ist in einer Bibliografie [NORDION 2013] zusammengefasst.

Die Mikrosphären der Firma SIRTEX nutzen die Fähigkeit von Epoxidharzkugeln, eine gewisse Menge von Y-90 an der Oberfläche ionisch zu binden.

SIRTEX hat die Technologie der Herstellung und Anwendung von radioaktiven Epoxidharzkugeln offengelegt (WO 02/34300 A1; US 2007/0253898 A1, US 2010/0215571 A1).

[SIRTEX 2013] enthält eine Bibliographie der Veröffentlichungen zur Anwendung von SirSpheres®.

SIRTEX hat ein Patent offengelegt, das sich auf die Erzeugung von radioaktiven Mikrosphären aus Glas bezieht (US 6,998,105). Es wird beschrieben, wie durch eine veränderte Mischung der Glasschmelze das Gewicht der Vollkugeln verringert werden soll, wodurch der Nachteil der hohen Glasdichte beseitigt wird. Eine erreichbare Dichte von weniger als 2,5 g/cm³ wird angegeben, wogegen die NORDION-Mikrosphären eine Dichte von größer 3 g/cm³ besitzen. Das nach SIRTEX erreichbare Minimum der Dichte ist demnach kleiner als 2,2 g/cm³, minimal 2,13 g/cm³. Das Radionuklid Y-90 für die Beladung, das ebenso wie bei NORDION durch Neutronenaktivierung von Y-89 erzeugt wird, ist an der Oberfläche von nichtporösem Glas fixiert. Die Erfindung verringert jedoch die Bildung der unerwünschten Radionuklide bei der Neutronenaktivierung nicht prinzipiell.

Es sind außer den NORDION-Vollglas-Mikrosphären (Therasphere®) und den SIRTEX-Epoxidharz-Mikrosphären (SIR-Spheres®) keine radioaktiven Mikrosphären weiterer Hersteller bekannt.

In EP 0210875 A3 (Theragenics Corporation, USA) wird ein Applikationssystem offengelegt, mit dem die Mikrosphären in den vaskulären Tumor eingeschwemmt werden können. Dieses System wird derzeit von der Firma NORDION weltweit genutzt.

Ein weiteres Applikationssystem wird in US 4,745,907 (Nuclear Medicine Inc., USA) offenbart, mit dem kleine radioaktive Partikel, wie z.B. Mikrosphären, in Lebertumore befördert werden sollen.

In der internationalen Patentanmeldung WO 2011/008939 A2 werden Mikrosphären aus porösem Glas im Durchmesserbereich von 20 bis 50 µm offenbart, die mit einem Biopolymer beladen sind. In einigen Ausführungsformen wird das Glas mit einem Radioisotop gedopt, welches aber ins Glas und nicht in die Poren eingelagert ist.

In der internationalen Patentanmeldung WO 02/34298 A1 werden Mikrosphären aus Glas im Durchmesserbereich von 20 bis 50 µm offenbart, welche Radionuklide aufweisen. Das Glas weist eine Dichte von weniger als 2,2 g/cm³ auf. Jedoch wird nicht-poröses Glas verwendet.

In der internationalen Patentanmeldung WO 99/51278 A1 werden Mikrosphären aus porösem und auch aus nicht porösem, anorganischen Material offenbart, wie auch Glas. Der Durchmesserbereich liegt zwischen 0,050 und 5.000 µm. In einigen Ausführungsformen sind die Mikrosphären mit einem Radioisotop beladen, das in die Poren eingelagert ist.

Eine Zielstellung der Erfindung ist es, radioaktive Mikrosphären für die Behandlung und Diagnostik von vaskulär versorgten Tumoren, insbesondere Lebertumoren zu erzeugen. Dabei ist das Radionuklid mit der Mikrosphäre so verbunden, dass kein Ausschwemmen in den Tumor erfolgt und nur die ionisierende Strahlung zur Behandlung des tumorösen Gewebes austritt. Eine weitere Zielstellung ist, Mikrosphären aus Glas zu erzeugen ohne radiochemische Verunreinigungen und mit einem Gewicht, das ähnlich oder kleiner als das von Epoxidharz-Mikrosphären gleicher Größe ist. Eine weitere Zielstellung ist die Beladung der Mikrosphären mit Radionukliden mit üblichen physikalisch-chemischen Verfahren und Geräten in Radionuklidlaboratorien ohne Einsatz von Kernreaktoren zur Neutronenaktivierung. Eine Option dieser Zielstellung ist es, die einfache Beladungstechnologie sehr einfach zu halten für eine Ausführbarkeit in radiopharmazeutischen oder chemischen Speziallabors von Kliniken. Dies wird Kliniken in die Lage versetzen, kurzfristig Patienten zu versorgen. Eine weitere Zielstellung ist die Beladungsoption von Mikrosphären für die nuklearmedizinische Diagnostik mit solchen Radionukliden, die vorwiegend als Photonenstrahlern wirken und eine exakte diagnostische Therapievorbereitung und Verlaufskontrolle gewährleisten. Eine Mehrfachbeladung der Mikrosphären mit therapeutischen und diagnostischen Radionukliden ist eine weitere Zielstellung der Erfindung.

Die Zielstellungen der Erfindung werden erreicht durch: mikroskopisch kleinem, nanoporösem Glas mit Kugelgeometrie im Durchmesserbereich von 25 bis 60 µm, beladen mit einem Radionuklid höchster radiochemischer Reinheit, das in die Nanoporen eingelagert ist, und bei dem zur Vermeidung des Ausschwemmens und Herauslösens des Radionuklides während der Therapie das Radionuklid bei der Herstellung in eine im Blut unlösliche Verbindung überführt wird. Die Größe der Mikrosphären liegt physiologisch bedingt zwischen 25 und 60 µm. Die Porengrößen der Mikrosphären liegen im Bereich von 5 bis 400 nm. Die Porenstruktur kann zwischen 30 und 90 Volumenprozent einer Mikrosphäre einnehmen. Die dadurch für die Beladung bereitgestellte, innere Oberfläche des nanoporösen Glases beträgt ein Vielfaches der äußeren Oberfläche und liegt zwischen 30 und 500 m²/g. Das nanoporöse Glas hat eine effektive (scheinbare) Dichte, die vom Porengehalt des Glases abhängt. Ein hoher Porengehalt bewirkt in wässrigen Systemen, dass sich die effektive Dichte der Mikrosphären der Dichte des wässrigen Systems annähert aber etwas darüber bleibt. Durch diese Eigenschaft des nanoporösen Glases kann eine effektive Dichte der Mikrosphären von kleiner 2,2 g/cm³, was der minimalen Dichte von reinem, nichtporösen Siliziumdioxid entspricht, erzielt werden. Vorzugsweise wird eine effektive Dichte der Mikrosphären während der Strahlentherapie von weniger als 1,5 g/cm³ erreicht.

Die nanoporösen Mikrosphären nehmen das Radionuklid bei der Beladung auf und behalten es nach einer Fixierungsbehandlung während der Therapie in den Poren. Die Beladung und Fixierung von einem oder mehreren Radionukliden kann mit gewöhnlichen, labortechnisch verfügbaren, chemischen und physikalischen Verfahren und Geräten nach Stand der Technik vorgenommen werden. Durch die kommerzielle Verfügbarkeit von hochreinen Radionukliden für die Beladung, wie bei Y-90 und In-111 der Fall, wird zur Herstellung der Mikrosphären keine Neutronenaktivierung gebraucht. Vorzugsweise wird Y-90 als therapeutisches Radionuklid eingesetzt. Diagnostische Radionuklide werden ausgewählt aus der Gruppe Indium-111 (In-111), Gallium-68 (Ga-68). Hochreines Y-90 und In-111 kann kommerziell beispielsweise von der Firma Perkin-Elmer (Kanada) bezogen werden. Ga-68 wird aus Radionuklidgeneratoren gewonnen. Nanoporöses Glas und die Art seiner Herstellung ist bekannt und Stand der Technik. Das Produkt und die Herstellung wird beispielsweise beschrieben in: DD 250 471 A1; DD 143 898 A1; DE 196 33 257 C1 und DE 410 26 35 A1 (VitraBio GmbH, Steinach, Deutschland). Die Beladung der Mikrosphären mit dem Radionuklid, das sich in einer chemischen Lösung befindet, wird mittels Inkubation in das oberflächenoffene Porensystem erreicht. In einem anschließenden Schritt wird die Lösung im Porensystem eingetrocknet und die chemische Verbindung calciniert. Dafür kommen vorzugweise thermische Behandlungen der inkubierten Mikrosphären mit der erforderlichen Zersetzungstemperatur der chemischen Verbindung des Radionuklides zur Anwendung. Andere Verfahren wie die Anwendung von Mikrowellen oder Licht sind optional einsetzbar bei geeigneten chemischen Verbindungen. Das Radionuklid wird in diesem Prozess im Porenvolumen vorzugsweise in sein Oxid überführt, was sich im Porenvolumen an den inneren Oberflächen ablagert, wogegen die gasförmigen Zersetzungsprodukte entweichen oder nichtgasförmige mit geeigneten Lösungsmitteln ausgewaschen werden. Einige Oxide von Radionukliden werden durch Bestandteile des Blutes gelöst und bedingen damit eventuell ein unerwünschtes Ausschwemmen des Radionuklides. Solche Oxide, beispielsweise Yttriumoxid, werden in einem weiteren Schritt in eine andere, im Blut weitaus geringer lösliche bis unlösliche Verbindung umgewandelt. Die Umwandlung wird dabei beispielsweise mit dem Zusatz von Säuren wie Flusssäure, Oxalsäure, schweflige Säure, Schwefelsäure oder Phosphorsäure in sehr geringer Konzentration vorgenommen. In einem weiteren Schritt werden das isolierte Radionuklid, das Oxid bzw. die optional erhaltenen, schwerlöslichen Verbindungen des Radionuklids thermisch auf dem Glas eingebrannt, was dann das Ausschwemmen quantitativ verhindert. Das geschieht bei Temperaturen unterhalb der Zersetzungstemperatur der Verbindungen. In einer Option wird das beispielsweise Yttriumoxid durch eine Hochtemperaturbehandlung der Mikrosphären bei Erhalt der Porenstruktur in die Glasoberfläche chemisch eingebunden. Zusätzliche Schritte zur Oberflächenveredelung der Mikrosphären sind optional. Beispielsweise werden durch eine Hydrophobierung die Suspendierbarkeit der Mikrosphären und das mechanische Fließverhalten bei der vaskulären Applikation verbessert. Eine weitere Option ist die gleichzeitige Beladung mit zwei verschieden Radionukliden, eines therapeutischen und eines diagnostischen. Dazu werden Radionuklide verwendet, die ähnliches chemische und physikalische Eigenschaften bezüglich der Herstellungsschritte aufweisen wie beispielsweise Y-90 mit In-111 oder Y-90 mit Ga-67 und Ga-68. Für den Beladungsprozess sind auch Verbindungen des Radionuklides geeignet, die sich nur in organischen Lösungsmitteln lösen lassen. Die Fixierung erfolgt dabei durch Verdampfen des Lösungsmittels und Einbrennen der Verbindung innerhalb der nanoporösen Struktur. Eine weitere Option des Beladungsprozesses ist dessen Durchführung durch den klinischen Anwender (z.B. im klinischen Radiopharmaziezentrum) selbst. Dieser bekommt die Ausgangsmaterialien übergeben und verfährt nach den vorgegebenen Beladungsschritten in seinem eigenen Labor (Kitlösung). Die Tumorbehandlung mit der SIRT wird durch die Injektion einer sehr hohen Anzahl von beladenen Mikrosphären in die vaskuläre Versorgung des Tumors bewirkt. Die Mikrosphären werden in den Arterien des Tumors wegen ihres zu großen Durchmessers gestoppt. Durch die eintretende Radioembolisation wird der Tumor therapiert. Die radioaktive Beladung der Gesamtzahl der Mikrosphären für einen Tumorherd wird so hoch gewählt, dass eine Strahlendosis von 80 bis 150 Gray im Tumor deponiert wird. Die Zahl der Mikrosphären in einer Tumordosis kann durch die sehr hohe Beladungsvariabilität von nanoporösen Mikrosphären von weniger als 1 Million bis hin zu mehreren Millionen variiert werden, was mit den bisherigen Mikrosphären nicht erreicht werden kann. Dadurch werden neue therapeutische Ansätze möglich. Für Tumore mit einem Durchmesser von einigen Zentimetern wird die Anzahl von Mikrosphären pro Tumor zwischen ein und vier Millionen gewählt. Durch das unkomplizierte Beladungs- und Fixierungsverfahren wird die obengenannte Kitlösung für die Herstellung im klinischen Umfeld möglich. Die Möglichkeit der Doppelbeladung mit therapeutischen und diagnostischen Radionukliden erfüllt die Anforderungen der Strahlenmedizin nach einer Verlaufskontrolle während und nach der Therapie, was die bisherigen Mikrosphären nicht leisten können.

### Ausführungsbeispiel 1:

Die therapeutische Planung basierend auf der radiologischen Bewertung der Größe des Lebertumors sieht für die Katheterapplikation Y-90-Aktivitäten von 20 GBq und eine Anzahl von vier Millionen Mikrosphären vor. Zur Kompensation des radioaktiven Zerfalls während der Herstellung und der Logistik wird eine Beladungsaktivität von 20 GBq gewählt. Y-90 wird als Nitrat in salpetersaurer Lösung eingesetzt. Die Porosität der Mikrosphären beträgt 75%. Die effektive Dichte in wässrigen Lösungen beträgt dadurch 1,4 g/cm³. Der Durchmesser der Mikrosphären beträgt im Mittel 30 µm.

Die Fertigung erfolgt patientenspezifisch auf Anforderung des Onkologen, d.h., die Patientendosis wird in diesem Beispiel nur für einen einzelnen Patienten gefertigt.

Folgende Schritte werden nacheinander ausgeführt:
- Auswiegen von 38 mg (ca. 76 µl) Mikrosphären, entspricht der benötigten Anzahl von 4 Millionen,
- Dreifaches Waschen in destilliertem Wasser und anschließendes Trocknen der Mikrosphären bei 105°C,
- Herstellung der radioaktiven Beladelösung aus 20 GBq Y-90-Nitrat in 60 µl in 0,05 molarer HNO₃ (entspricht etwa dem Porenvolumen der Mikrosphären),
- Einfüllen der Mikrosphären in ein Eppendorf-Röhrchen und Auftropfen der 60 µl Y-90-Beladelösung,
- Einstellen des nichtverschlossenen Eppendorf-Röhrchens in einen Exsikkator und evakuieren bis 10 mbar für etwa 1 Stunde,
- Trocknen des unverschlossenen Eppendorf-Röhrchens im Trockenschrank bei 60 bis 105°C langsam ansteigend,
- Umfüllen der Mikrosphären in ein Porzellanschiffchen und langsames Erhitzen im Brennofen auf 600°C und für eine Stunde halten,
- nach Abkühlen Umfüllen der Mikrosphären in ein neues Eppendorf-Röhrchen und dreifaches Waschen in je 1 ml dest. Wasser mit Zentrifugation, mit anschließendem Trocknen im Wärmeschrank bei 80°C für 1 Stunde,
- Auftropfen von 60 µl 0,005 molarer HF, Inkubation im Exsikkator bei 10 mbar für 10 Minuten und anschließender Reaktionszeit von 30 Minuten im Wärmeschrank bei 30°C im Eppendorf-Röhrchen,
- Trocknen im Wärmeschrank bei 105°C zum vollständigen Austreiben der nicht umgesetzten HF,
- Umsetzen der Mikrosphären in Porzellanschälchen und Einbrennen des Yttriumfluorid bei 750°C,
- Umsetzen der Mikrosphären in Eppendorf-Röhrchen,
- Dreifaches Waschen in je 1 ml dest. Wasser mit anschließender Abtrennung des Waschwasser durch Zentrifugation,
- Vermessen der Beladungsaktivität in einer Ionisationskammer,
- Umfüllen der Mikrosphären durch Aufnahme mit 1,5 ml physiologischer Kochsalzlösung in ein sterilisierbares V-Vial (3 ml) und Verschließen mit sterilisierbarem Krimpverschluss,
- Autoklavieren.

Nach dem Autoklavierprozess sind die Mikrosphären nach etwa 6 Tagen einsetzbar, nachdem sie durch radioaktiven Zerfall die verordnete Aktivität erreicht haben. Vor der Applikation in den Tumor des Patienten wird eine weitere messtechnische Aktivitätskontrolle eines Medizinphysikers vorgenommen.

### Ausführungsbeispiel 2:

Zur Vorbereitung der Tumorbehandlung ist die Bestimmung des Shunts notwendig, der klärt, ob der Patient für die Therapie des Lebertumors mit Mikrosphären geeignet ist. Der Shunt stellt das Maß für den Verlust von Mikrosphären dar, die nicht im Lebertumor verbleiben und sich unerwünscht in anderen Körperbereichen ablagern würden. Die diagnostische Darstellung mittels nuklearmedizinischer Methoden kann mit dem Radionuklid In-111 ausgeführt werden. Es wird eine Beladungsaktivität von 200 MBq gewählt. Die Anzahl der Mikrosphären wird auf 400.000 eingewogen. Die weiteren Schritte sind wie in Ausführungsbeispiel 1 beschrieben auszuführen. Die eingesetzte Menge an Mikrosphären ist entsprechend auf 3,8 mg reduziert und die Mengen an HNO₃ und HF auf 6 µl angepasst. Als unlösliche Verbindung wird Indiumfluorid gebildet und eingebrannt.

## Patentansprüche

1. Strahlentherapeutisches Erzeugnis bestehend aus
Mikrosphären aus nanoporösem Glas mit kugelförmiger Geometrie im Durchmesserbereich von 25 bis 60 µm, vorzugsweise 25 bis 40 µm, beladen mit einem Radionuklid, das in die Nanoporen eingelagert ist, und bei dem zur Vermeidung des Ausschwemmens und Herauslösens des Radionuklides während der Therapie das Radionuklid bei der Herstellung in eine im Blut unlösliche Verbindung überführt wird.

2. Strahlentherapeutisches Erzeugnis nach Anspruch 1
mit einer effektiven (scheinbaren) Dichte kleiner als 2,2 g/cm³, vorzugsweise kleiner als 1,5 g/cm³.

3. Strahlentherapeutisches Erzeugnis nach Anspruch 1,
wobei die Mikrosphären eine innere Oberfläche des Porensystems von wenigstens 40 m²/g haben.

4. Strahlentherapeutisches Erzeugnis nach Anspruch 1,
wobei der Durchmesser der Nanoporen aus dem Bereich von 5 bis 400 nm gewählt wird.

5. Strahlentherapeutisches Erzeugnis nach Anspruch 1 bis 4
beladen mit einem, optional zwei unterschiedlichen Radionukliden mit hoher radiochemischer Reinheit, wobei das erste Radionuklid eine therapeutische Wirkung erzielt, vorzugsweise Y-90, und das zweite Radionuklid vorzugsweise eine nuklearmedizinisch-diagnostische Eigenschaften hat, vorzugsweise In-111 oder Ga-68.

6. Strahlentherapeutisches Erzeugnis nach Anspruch 1 bis 5,
bei dem die unlösliche Verbindung durch Einbrennen an den Oberflächen der Nanoporen physikalisch fixiert wird.

7. Strahlentherapeutisches Erzeugnis nach Anspruch 1 bis 5,
bei dem die unlösliche Verbindung chemisch an den Oberflächen der Nanoporen gebunden ist.

8. Strahlendiagnostisches Erzeugnis nach Anspruch 1 bis 4,
ausschließlich beladen mit einem nuklearmedizinisch-diagnostischen Radionuklid, vorzugsweise In-111 oder Ga-68.

## Claims

1. Radiotherapy product consisting of microspheres made of nano-porous glass with a spherical geometry at a diameter ranging from 25 to 60 µm, preferably 25 to 40 µm, loaded with a radionuclide that is embedded in the nanopores, and where the radionuclide is converted during production to an insoluble compound within the blood in order to avoid the flushing or dissolving out of the radionuclide.

2. Radiotherapy product according to Claim 1 with an effective (apparent) density less than 2.2 g/cm³, preferably less than 1.5 g/cm³.

3. Radiotherapy product according to Claim 1, wherein the microspheres have an inner surface of the pore system of at least 40 m²/g.

4. Radiotherapy product according to Claim 1, wherein the diameter of the nanopores is selected from the range from 5 to 400 nm.

5. Radiotherapy product according to Claims 1 to 4, loaded with one, and optionally two different radionuclides with a high radiochemical purity, wherein the first radionuclide achieves a therapeutic effect, preferably Y-90, and the second radionuclide preferably has nuclear-medicine-diagnostic characteristics, preferably In-111 or Ga-68.

6. Radiotherapy product according to Claim 1 to 5, where the insoluble connection is physically fixated by means of burning onto the surfaces of the nanopores.

7. Radiotherapy product according to Claim 1 to 5, where the insoluble connection is chemically bound to the surfaces of the nanopores.

8. Radiotherapy product according to Claims 1 to 4, only loaded with a nuclear-medicine-diagnostic radionuclide, preferably In-111 or Ga-68.

## Revendications

1. Article de thérapie aux rayons consistant en microsphères en verre nanoporeux d'une géométrie en forme de billes dans une fourchette de diamètre de 25 à 60 µm, de préférence 25 à 40 µm, chargé d'un radionucléide qui est intégré dans les nanopores, et dans lequel, pour éviter l'élimination et la dissolution du radionucléide pendant la thérapie, le radionucléide est transformé lors de la fabrication en un composé insoluble dans le sens.

2. Article de thérapie aux rayons selon la revendication 1, ayant une densité effective (apparente) inférieure à 2,2 g/cm3, de préférence inférieure à 1,5 g/cm3.

3. Article de thérapie aux rayons selon la revendication 1, dans lequel les microsphères ont une surface intérieure de système poreux de moins de 40 m²/g.

4. Article de thérapie aux rayons selon la revendication 1, dans lequel le diamètre des nanopores est sélectionné dans la fourchette de 5 à 400 nm.

5. Article de thérapie aux rayons selon les revendications 1 à 4, chargé d'un, en option de deux, radionucléides différents à haute pureté radiochimique, le premier radionucléide visant un effet thérapeutique, de préférence Y-90, et le second radionucléide ayant de préférence une propriété de diagnostic en médecine nucléaire, de préférence In-111 ou Ga-68.

6. Article de thérapie aux rayons selon les revendications 1 à 5, dans lequel le composé insoluble est fixé physiquement par brûlage aux surfaces des nanopores.

7. Article de thérapie aux rayons selon les revendications 1 à 5, dans lequel le composé insoluble est lié chimiquement aux surfaces des nanopores.

8. Article de thérapie aux rayons selon les revendications 1 à 4, chargé exclusivement d'un radionucléide de diagnostic en médecine nucléaire, de préférence In-111 ou Ga-68.
